# EUROPEAN PATENT APPLICATION

(11) **EP 1 674 094 A1**
(43) Date of publication of application: **28.06.2006**
(21) Application number: 03754128.1
(22) Date of filing: 15.10.2003
(51) Int. Cl.: A61K 31/203, A61K 9/51, A61K 9/06, A61K 9/70, A61K 8/18, A61K 8/99, A61P 17/00, A61P 3/02

(54) **COMPOSITION CONTAINING RETINOIC ACID NANOPARTICLES COATED WITH POLYVALENT METAL INORGANIC SALT**

(71) Applicant: LTT Bio-Pharma Co., Ltd., Tokyo 105-6201 (JP)
(72) Inventor: YAMAGUCHI, Yoko, Ashigarakami-gun, Kanagawa 258-0022 (JP); IGARASHI, Rie, Kawasaki-shi, Kanagawa 214-0036 (JP); MIZUSHIMA, Yutaka, Minato-ku, Tokyo 106-0032 (JP); TAKENAGA, Mitsuko, Kawasaki-shi, Kanagawa 216-0015 (JP); NAKAMURA, Natsumi, Kawasaki-shi, Kanagawa 216-0005 (JP)
(74) Representative: Albrecht, Thomas
(86) International application number: PCT/JP2003/013181
(87) International publication number: WO 2005/037268

(57) **Abstract**

A composition contains nanoparticles of retinoic acid as an active ingredient. The nanoparticles have reduced irritancy of retinoic acid and are suitable for subcutaneous or intravenous administration, as well as for use in sustained-release preparation. The high skin permeability of the nanoparticles makes the composition suitable for use in pharmaceutical or non-pharmaceutical external preparations or cosmetics intended for skin application.

The retinoic acid nanoparticles of the present invention are coated with an inorganic salt of polyvalent metal and can be dissolved in water to make a stable clear solution that can be formulated into injectable preparations for subcutaneous and intravenous administration.

The polyvalent metal inorganic salt coating helps reduce the irritancy of retinoic acid, so that the nanoparticles do not cause inflammation or tumor formation at the site of application.

The inorganic salt of polyvalent metal may be calcium carbonate, zinc carbonate or calcium phosphate.

## Description

### TECHNICAL FIELD

The present invention relates to a composition containing as an active ingredient retinoic acid nanoparticles coated with an inorganic salt of polyvalent metal, and particularly to a composition containing retinoic acid nanoparticles coated with calcium carbonate, zinc carbonate, or calcium phosphate. More particularly, the present invention relates to oral preparations, non-oral preparations, external preparations, and cosmetics that contain as an active ingredient the retinoic nanoparticles coated with calcium carbonate, zinc carbonate, or calcium phosphate.

### TECHNICAL BACKGROUND

Retinoic acid, a liposoluble vitamin A acid, has recently attracted much attention for its ability to induce differentiation of embryonic stem (ES) cells and various other undifferentiated cells. Retinoic acid has been clinically used as a cure for acute promyuelocytic leukemia.

However, retinoic acid has irritancy due to carboxyl groups present in the compound and, when subcutaneously administered, causes inflammation or tumor formation at the site of injection. Moreover, the high solubility of retinoic acid in lipids makes it difficult to formulate the compound into injections. Accordingly, various drug delivery systems (DDSs) have been proposed that are designed for sustained-release or targeted delivery of retinoic acid (See, for example, C. S. Cho, K. Y. Cho, I. K. Park, S. H. Kim, T. Sugawara, M. Uchiyama & T. Araike: *"Receptor-mediated delivery of all trans-retinoic acid to hepatocyte using poly(L-lactic acid) nanoparticles coated with galactose-carrying polystylene",* J.

### Control Release, 2001 Nov. 9:77(1-2), 7-15).

An injection has also been proposed that uses biodegradable polymer (See, for example, G. G. Giordano, M. FD. Refojo & M. H. Arroyo: *"Sustained delivery of retinoic acid from microsphers of biodegradable polymer in PVR", Invest. Ophthalmol. Vis., 1993 Aug. 34(9): 274-2745).*

Retinoic acid also has an ability to promote the growth of epithelial cells and, thus, the possibility of its use in cosmetics has been examined: The compound is expected to act to eliminate skin wrinkles and act as a skin-vitalizing or anti-aging agent (Japanese Patent Laid-open Publication No. Hei 09-503499). Nonetheless, the strong irritancy of retinoic acid, a common property of carboxylic acids, causes inflammation and other skin problems, making the compound unsuitable for use in cosmetics.

In an attempt to address these problems, the present inventors have previously proposed retinoic acid-containing nanoparticles that can be delivered subcutaneously or intravenously for sustained-release of the active ingredient. When applied to skin, the nanoparticles can elicit the advantageous effects of retinoic acid (See, for example, Japanese Patent Laid-Open Publication No. 2003-172493; *Journal of Pharmaceutical Science and Technology, Vol.62 Mar. 2002, Supplement,* The Academy of Pharmaceutical Science and Technology, Japan, Abstracts of lectures of 17th annual meeting; *Drug Delivery System (DDS), Vol. 18, No. 3 May.:221 (2003); 29th Annual Meeting of the Controlled Release Society in Collaboration with the Korean Society for Biomaterials; Final Program July 20-25* (*2002)*).

The previously proposed retinoic acid-containing nanoparticles are prepared in the following manner: Retinoic acid dissolved in a small amount of a polar solvent is dispersed in an alkali-containing water. To this dispersion, a nonionic surfactant is added to form mixed micelles, to which a salt of divalent metal is added, followed by a salt that can form negative divalent ion. This gives the desired product.

The retinoic acid-containing nanoparticles so prepared comprise particles that have a coating of a metal compound deposited on the surface thereof. For example, when the salt of divalent metal is calcium chloride and the salt that can form negative divalent ion is sodium carbonate, a coating of calcium carbonate is deposited on the surface of nanoparticles.

The retinoic acid-containing nanoparticles previously provided by the present inventors are prepared by taking advantage of the amphipathic property of retinoic acid. Specifically, retinoic acid is first dispersed in an aqueous solution to form spherical micelles having negatively charged surface. A nonionic surfactant and then calcium chloride are added to allow calcium ion (Ca²⁺) to adsorb onto the negatively charged micelle surface. This prevents aggregation and subsequent precipitation of retinoic acid micelles and gives spherical or oval micelles covered with calcium ions. Sodium carbonate is then added to allow carbonate ion (CO₃²⁻) to adsorb onto (bind to) the calcium ion-coated micelle surface and thus completely neutralize the surface charge of the micelles. As a result, calcium carbonate coating is deposited on the surface of the retinoic acid micelles, thus giving the desired calcium carbonate-coated retinoic acid nanoparticles.

As opposed to calcium carbonate crystals obtained by the precipitation method or the uniform precipitation method, which are substantially water-insoluble crystals commonly known as calcite, the calcium carbonate deposited on the spherical or oval micelle surface by the above-described process is not likely to form hard crystals, but rather has a glass-like amorphous structure or metastable vaterite structure. If the calcium carbonate coating has amorphous structure, which unlike hard crystal structure, has a high solubility in water and is highly biodegradable, the coating is readily decomposed. Similarly, the coating formed as a vaterite is readily biodegraded since vaterite has a higher solubility in water than the other crystalline forms of calcium carbonate: calcite and aragonite.

Thus, the calcium carbonate-coated retinoic acid nanoparticles obtained by the above-describe process, when administered to a living body, have a sustained effect as the calcium carbonate layer on the micelle surface is degraded to release retinoic acid contained in the micelles.

Aside from calcium carbonate, other biocompatible inorganic salts of polyvalent metals, such as zinc carbonate and calcium phosphate, may be used to coat the surface of the micelles containing retinoic acid to achieve the same effect.

One problem is that the retinoic acid nanoparticles coated with calcium carbonate or other inorganic salts of polyvalent metal have a varying particle size (diameter) of 5 to 1000 nm and it has been considered difficult to effectively prepare nanoparticles of desired size: It is preferred that the nanoparticles are very small, specifically approximately 5 to 300 nm in size, for subcutaneous, intravenous, or topical application (transdermal application) of retinoic acid.

Accordingly, it is an object of the present invention to provide a composition that contains as an active ingredient approximately 5 to 300 nm very small retinoic acid nanoparticles coated with an inorganic salt of polyvalent metal, such as calcium carbonate, and that can be used in preparations for subcutaneous and intravenous administration or in external preparations and cosmetics for skin application.

In an effort to achieve this object, the present inventors have found that by adjusting the molar ratios of the metal halide and the alkali metal carbonate or alkali metal phosphate added during deposition of the coating of polyvalent metal inorganic salt on the surface of retinoic acid micelles, the coated retinoic acid nanoparticles having an average particle of approximately 5 to 300 nm can be obtained. It is this discovery that led to the present invention.

### DISCLOSURE OF THE INVENTION

Accordingly, basic embodiments of the present invention comprise the following:
(1) A composition comprising as an active ingredient retinoic acid nanoparticles that comprise micelles of retinoic acid coated with an inorganic salt of polyvalent metal and have an average particle size of 5 to 300 nm.
(2) The composition according to (1) above, wherein a coating of the polyvalent metal inorganic salt of the retinoic acid nanoparticles to serve as the active ingredient is calcium carbonate, zinc carbonate, or calcium phosphate.
(3) The composition according to (1) or (2) above, wherein the retinoic acid nanoparticles to serve as the active ingredient is obtained by:
   dispersing retinoic acid dissolved in a lower alcohol in an aqueous alkali solution;
   adding a nonionic surfactant to the dispersion to form a mixed micelle;
   adding to the micelle a halide or acetate of divalent metal along with a carbonate or phosphate of alkali metal so that a molar ratio of the former to the latter is 1:0 to 1:1.0, thereby depositing a coating of the inorganic salt of the polyvalent metal on a surface of the micelle; and
   adjusting an average particle size of the resulting nanoparticles to 5 to 300 nm.
(4) The composition according to (1), (2) or (3) above,
   wherein the active ingredient is retinoic acid nanoparticles having an average particle size of 5 to 300 nm and coated with calcium carbonate.
(5) The composition according to (1), (2) or (3) above,
   wherein the active ingredient is retinoic acid nanoparticles having an average particle size of 5 to 300 nm and coated with zinc carbonate.
(6) The composition according to (1), (2) or (3) above,
   wherein the active ingredient is retinoic acid nanoparticles having an average particle size of 5 to 300 nm and coated with calcium phosphate.
(7) The composition according to (1) through (6) above for use as an oral preparation, a non-oral preparation, an external preparation, or a cosmetic.
(8) The composition according to (7) above, being a sustained-release composition.
   Thus, specific embodiments of the present invention comprises the following:
(9) A sustained-release preparation containing as an active ingredient retinoic acid nanoparticles having an average particle size of 5 to 300 nm and coated with calcium carbonate.
(10) An external preparation containing as an active ingredient retinoic acid nanoparticles having an average particle size of 5 to 300 nm and coated with calcium carbonate.
(11) A cosmetic containing retinoic acid nanoparticles having an average particle size of 5 to 300 nm and coated with calcium carbonate.
(12) A sustained-release preparation containing as an active ingredient retinoic acid nanoparticles having an average particle size of 5 to 300 nm and coated with zinc carbonate.
(13) An external preparation containing as an active ingredient retinoic acid nanoparticles having an average particle size of 5 to 300 nm and coated with zinc carbonate.
(14) A cosmetic containing retinoic acid nanoparticles having an average particle size of 5 to 300 nm and coated with zinc carbonate.
(15) A sustained-release preparation containing as an active ingredient retinoic acid nanoparticles having an average particle size of 5 to 300 nm and coated with calcium phosphate.
(16) An external preparation containing as an active ingredient retinoic acid nanoparticles having an average particle size of 5 to 300 nm and coated with calcium phosphate.
(17) A cosmetic containing retinoic acid nanoparticles having an average particle size of 5 to 300 nm and coated with calcium phosphate.

The retinoic acid nanoparticles coated with an inorganic salt of polyvalent metal to serve as the active ingredient of the composition provided by the present invention have a very small average particle size adjusted to the range of 5 to 300 nm.

Retinoic acid is a highly irritant and lipophilic compound and can thus cause inflammation and tumor formation at the site of application when subcutaneously administered. Moreover, the insolubility of retinoic acid in water makes it unsuitable for use in injections. On the other hand, the retinoic acid nanoparticles coated with an inorganic acid of polyvalent metal of the present invention can be dissolved in water to form a clear solution, which remains clear when left and can thus be formulated into injection preparations for subcutaneous and intravenous administration. The inorganic salt coating is biocompatible and helps reduce the irritancy of retinoic acid, so that the nanoparticles do not cause inflammation or tumor formation at the site of application.

In addition, when applied to skin as an external preparation, the nanoparticles of the present invention are percutaneously absorbed, yet cause no inflammation because of less irritancy. The nanoparticles then release retinoic acid in a sustained manner, acting to eliminate skin wrinkles and activate skin.

Retinoic acid is particularly effective when applied to skin: It promotes the growth of epithelial cells and facilitates the regeneration of the skin. Although these advantageous properties have led to the expectation that the compound can be used for the purposes of skin beauty and wrinkle elimination, its skin irritancy has prevented the use of retinoic acid in cosmetics. With the coating of inorganic salt of polyvalent metal, however, the nanoparticles of the present invention have significantly reduced irritancy. Moreover, the small average particle size of 5 to 300 nm helps improve the skin permeability of the particles and facilitates the diffusion of retinoic acid into blood, so that the blood level of retinoic acid quickly reaches the effective point and remains there for an extended period of time.

This increases the production of HB-epidermal growth factor (HB-EGF) and induces the production of hyaluronic acid, which otherwise is not produced in epidermis in a short time. As a result, the regeneration of the skin is significantly accelerated, as is the thickening of epidermis. Thus, the nanoparticles of the present invention are highly useful not only in cosmetics, but also in regenerative medicine.

### BRIEF DESCRIPTION OF THE DRAWINGS

Fig. 1 is a diagram showing ³H-thymidine uptake by melanoma cells stimulated by retinoic acid in accordance with Test Example 4.
Fig. 2 is a diagram showing the change in the blood level of retinoic acid when retinoic acid-CaCO₃ nanoparticles and retinoic acid micelles were subcutaneously administered to rats according to (1) of Test Example 5.
Fig. 3 is a photograph showing the site of application 10 days after the retinoic acid micelles (not formulated as nanoparticles) were subcutaneously administered to rats according to (1) of Test Example 5.
Fig. 4 is a photograph showing the site of application 10 days after the retinoic acid-CaCO₃ nanoparticles of the present invention were subcutaneously administered to rats according to (1) of Test Example 5.
Fig. 5 is a diagram showing the change in the blood level of retinoic acid when retinoic acid-CaCO₃ nanoparticles, retinoic acid-ZnCO₃ nanoparticles, and retinoic acid were mixed with a Vaseline base and were individually applied to the skin of mice according to (2) of Test Example 5.
Fig. 6 is a diagram showing a comparison of expression levels of HB-EGF mRNA according to Test Example 6.
Fig. 7 is a diagram showing the thickness of epidermis in mice administered different preparations according to Test Example 7.
Fig. 8 is a photograph of stained skin tissue (HE staining) of a non-treated group to serve as control in Test Example 7.
Fig. 9 is a photograph of stained skin tissue (HE staining) of a group administered a preparation containing retinoic acid alone in Test Example 7.
Fig. 10 is a photograph of stained skin tissue (HE staining) of a group administered a preparation containing retinoic acid-CaCO₃ nanoparticles in Test Example 7.
Fig. 11 is a photograph of stained skin tissue (HE staining) of a group administered a preparation containing retinoic acid-ZnCO₃ nanoparticles in Test Example 7.
Fig. 12 is a photograph of stained skin tissue (HE staining) of a group administered a preparation containing retinoic acid-Ca nanoparticles in Test Example 7.
Fig. 13 is a photograph of stained skin tissue (HE staining) of a group administered a preparation containing retinoic acid-Zn nanoparticles in Test Example 7.
Fig. 14 is a photograph of stained skin tissue (colloidal iron staining) of a group administered a preparation containing retinoic acid alone in Test Example 7.

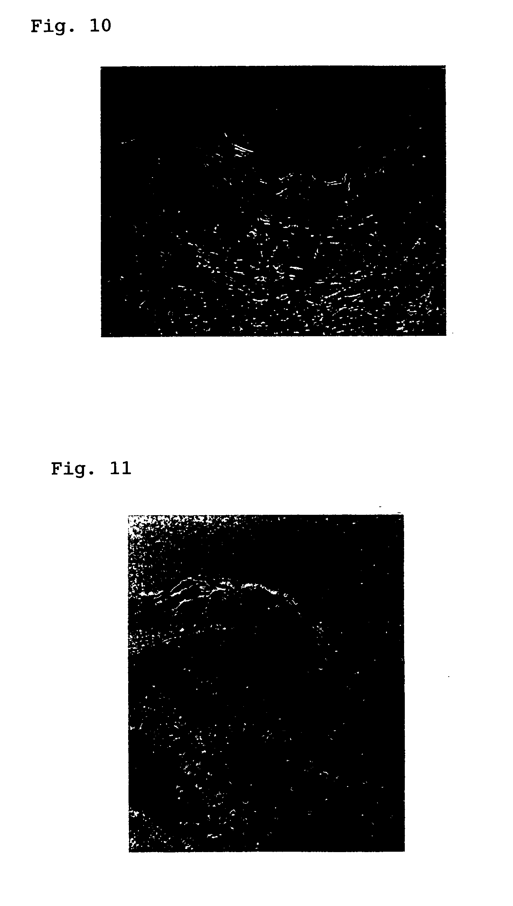

Fig. 10 is a photograph of stained skin tissue (colloidal iron staining) of a group administered a preparation containing retinoic acid-CaCO₃ nanoparticles in Test Example 7.

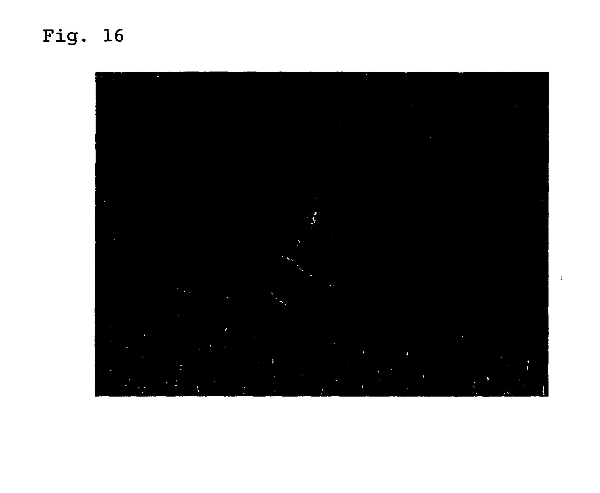

Fig. 16 is a photograph of the neck area of a hairless mouse prior to administration in Test Example 8.

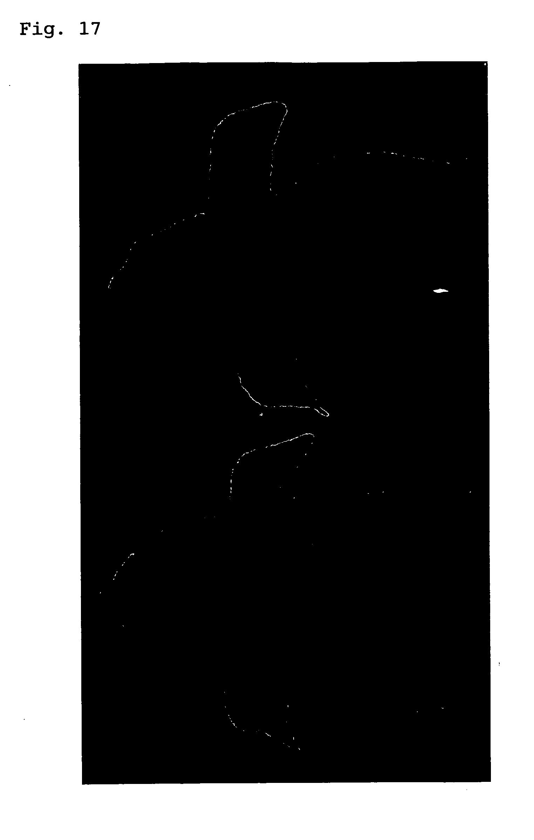

Fig. 17 is a photograph of the neck area of hairless mice after a 4-day application period of a preparation containing retinoic acid alone or a preparation containing retinoic acid-CaCO₃ nanoparticles of the present invention in Test Example 8.

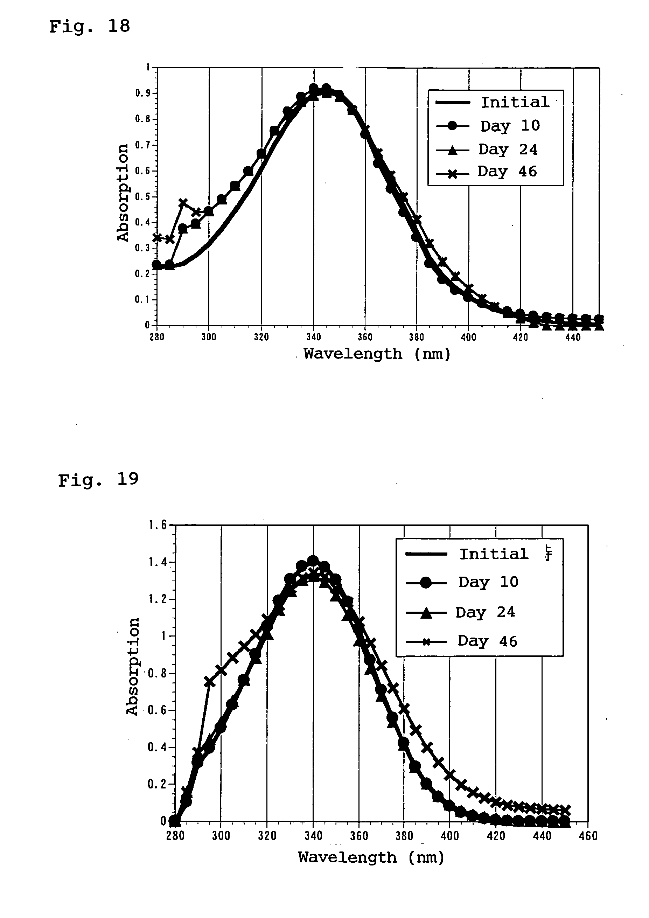

Fig. 18 is a graph showing the change in the peak absorption by a preparation containing retinoic acid alone in Test Example 9.
Fig. 19 is a graph showing the change in the peak absorption by an aqueous preparation containing retinoic acid-CaCO₃ nanoparticles in Test Example 9.

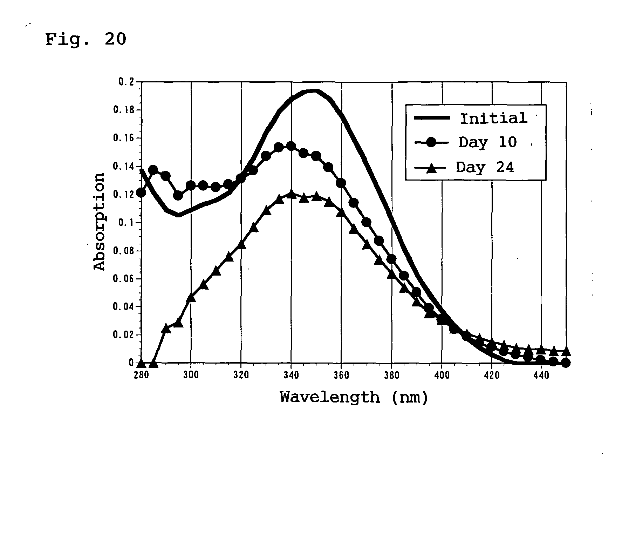

Fig. 20 is a graph showing the change in the peak absorption by a Vaseline-based preparation containing retinoic acid-CaCO₃ nanoparticles in Test Example 9.

Throughout the drawings, RA indicates retinoic acid; RA-CaCO₃ indicates retinoic acid-CaCO₃ nanoparticles; RA-ZnCO₃ indicates retinoic acid-ZnCO₃ nanoparticles; RA-Ca indicates retinoic acid-Ca nanoparticles; and RA-Zn indicates retinoic acid-Zn nanoparticles.

### BEST MODE FOR CARRYING OUT THE INVENTION

Retinoic acid for use in the present invention is all-trans retinoic acid, a compound involved in various physiological functions, including proper functioning of vision, auditory sense and reproductive functions, maintenance of skin and mucosa and suppression of cancer. All-trans retinoic acid has been clinically used in the treatment of acute promyelocytic leukemia (APL).

Specifically, the retinoic acid nanoparticles coated with an inorganic salt of a polyvalent metal are prepared as described below.

A lipophilic compound with carboxyl groups in its molecule, retinoic acid forms spherical micelles in an aqueous alkali solution, such as aqueous sodium hydroxide solution, containing a small amount of a lower alcohol. The surface of the micelle is negatively charged and readily adsorbs (binds to) divalent metal ion, such as calcium ion (Ca²⁺), replacing sodium ion. Since the divalent metal ion is more tightly adsorbed (bound) to the micelles than is the sodium ion, the micelles having the divalent metal ions adsorbed on them have more stable surface charge, so that they become insoluble in water and precipitate. The precipitated particles aggregate into large clusters.

To prevent aggregation of the charged particles, a nonionic surfactant, such as polyoxyethylene (20) sorbitan monooleate (Tween 80), is added along with retinoic acid. Tween 80, together with retinoic acid, forms mixed micelles that have polyoxyethylene chains sticking out from their surface. The presence of the hydrophilic polyoxyethylene chains on the micelle surface prevents the precipitation of the micelles when they adsorb (bind to) polyvalent metal ions.

A halide or acetate of a divalent metal, such as calcium chloride, is then added in a sufficiently large amount so that the divalent metal ions can adsorb onto the surface of the retinoic acid micelles. The divalent metal ions are more tightly adsorbed (bound) to the micelle surface than sodium ions and thus replace sodium ions on the micelle surface. The primarily adsorbed (bound) divalent metal ions cover the micelle surface to form spherical or oval micelles. A carbonate or phosphate of an alkali metal is then added to the system to allow carbonate ions (CO₃²⁻) or phosphate ions (PO₄²⁻) to adsorb onto the divalent ions on the still unneutralized micelle surface. These results in the deposition of a coating of an inorganic salt of polyvalent metal on the surface of the retinoic acid micelles, thus giving the desired retinoic acid nanoparticles coated with an inorganic salt of polyvalent metal.

The inorganic salt of a polyvalent metal that coats the nanoparticles of the present invention may be calcium carbonate, zinc carbonate or calcium phosphate, each a biocompatible salt.

Thus, the halide or acetate of divalent metal is calcium halide, zinc halide, calcium acetate or zinc acetate. Specific examples of the calcium halide and zinc halide include calcium chloride, calcium bromide, calcium fluoride, calcium iodide, zinc chloride, zinc bromide, zinc fluoride and zinc iodide.

Examples of the alkali metal carbonate or alkali metal phosphate include sodium carbonate, potassium carbonate, sodium phosphate and potassium phosphate.

The lower alcohol for use in the preparation of the nanoparticles may be methanol or ethanol.

Examples of the nonionic surfactant include polyoxyethylene (20) sorbitan monooleate (Tween 80), polyoxyethylene (20) sorbitan monolaurate (Tween 20), polyoxyethylene (20) sorbitan monostearate (Tween 60), polyoxyethylene (20) sorbitan monopalmitate (Tween 40), polyoxyethylene (20) sorbitan trioleate (Tween 85), polyoxyethylene (8) octylphenylether, polyoxyethylene (20) cholesterol ester, and polyoxyethylene hydrogenated castor oil.

While the coated retinoic acid nanoparticles prepared by the above-described method are very small, they have a wide particle size distribution ranging from about 10 to about 3000 nm (in diameter).

It is preferred that the retinoic acid nanoparticles have a very small size of about 5 to about 300 nm when they are intended for subcutaneous, intravenous or topical application (transdermal application). Thus, the size of the desired retinoic acid nanoparticles coated with an inorganic acid of polyvalent metal must be adjusted to about 5 to about 300 nm.

It has turned out that the size of the nanoparticles can be adjusted to the desired range by varying the amounts of the components for depositing the coating on the surface of the retinoic acid micelle, specifically by varying the molar ratio of the halide or acetate of divalent metal to the carbonate or phosphate of alkali metal, and by mechanically shaking the particles, for example, by means of ultrasonication.

Specifically, the coating of the polyvalent metal inorganic salt is deposited on the surface of the retinoic acid micelles by exchanging the negative charge imparted to the micelle surface in the alkali (i.e., sodium) solution for the divalent metal ion resulting from the halide or acetate of divalent metal and by neutralizing the negative charge with the carbonate ion (CO3²⁻) or phosphate ion (PO4²⁻) resulting from the carbonate or phosphate of alkali metal.

In particular, by adjusting the molar ratio of the halide or acetate of divalent metal to the carbonate or phosphate of alkali metal within the range of 1:0 to 1:1.0, the coating of polyvalent metal inorganic salt is deposited on the micelle surface and the average particle size of the resulting nanoparticles is adjusted within the range of 5 to 300 nm. If necessary, the particles may be mechanically shaken by for example ultrasonication.

If 1.0mol or more of the carbonate or phosphate of alkali metal is added per 1mol of the halide or acetate of divalent metal, the resulting particles will become excessively large in size though the micelle surface may be properly coated with the inorganic salt of polyvalent metal. As a result, the particles aggregate with each other, so that the nanoparticles with the desired average particle size can no longer be obtained even by ultrasonication.

Conversely, if the molar ratio of the halide or acetate of divalent metal to the carbonate or phosphate of alkali metal is within the range of 1:0 to 1:1.0, then not only are the micelles properly coated with the inorganic salt of polyvalent metal, but the resulting nanoparticles have an average particle size of 5 to 300 nm.

The resulting nanoparticles may form aggregates. It has proven that such aggregates can be mechanically shaken by, for example, ultrasonication to obtain nanoparticles highly uniform in size. Thus, such aggregates are also encompassed by the scope of the present invention.

The so prepared retinoic acid nanoparticles coated with an inorganic salt of polyvalent metal provided in accordance with the present invention can be dissolved in water to form a stable clear solution and causes less irritancy since retinoic acid is coated with the polyvalent metal inorganic salt. The nanoparticles can thus be formulated into injection preparations for subcutaneous or intravenous administration. In addition, the nanoparticles do not cause inflammation or tumor formation at the site of application.

Moreover, when applied to skin as an external preparation, the nanoparticles of the present invention are percutaneously absorbed, yet cause no inflammation because of less irritancy. The nanoparticles then release retinoic acid in a sustained manner, acting to eliminate skin wrinkles and activate skin.

Containing as an active ingredient the above-described retinoic acid nanoparticles coated with an inorganic salt of polyvalent metal, the composition provided in accordance with the present invention is suitable for use in oral preparations, non-oral preparations, external preparations, and cosmetics. The composition is capable of sustained release of retinoic acid.

Examples of such oral preparations include tablets, capsules, granules, fine powders, and syrups. Examples of non-oral preparations include injections (such as subcutaneous and intravenous injection), intravenous drip infusions and eyedrops, as well as nasal or oral cavity preparations such as sprays and aerosols. Examples of external preparations include ointments, creams and poultices.

Any of these preparations can be prepared according to the Japanese Pharmacopoeia, General Rules for Preparations, and can be formulated with any suitable carriers, adjuvants, lubricants, fluidizing agents, disintegrating agents, binders, isotonic agents, and stabilizers and any other agents commonly used in the preparation of pharmaceutical compositions.

The retinoic acid nanoparticles coated with an inorganic acid of polyvalent metal to serve as the active ingredient of the composition of the present invention may be administered to a subject in any suitable dose. While the dose generally varies depending on factors such as sex, age, body weight, and symptoms of the patient, it is preferably such that the pharmacological activity of retinoic acid can be effectively exploited.

The composition of the present invention can be used as a treatment for ischemic diseases such as myocardial infarction, angina pectoris, and cerebral infarction.

The present invention also provides a variety of cosmetic products, including basic skin cares such as creams, emulsions, lotions, face cleansings, and facial packs, and make-up cosmetics such as lipsticks and foundations. The retinoic acid nanoparticles coated with an inorganic acid of polyvalent metal may be added to the cosmetic products in any suitable amount and may be used together with appropriate fragrances. Furthermore, the retinoic acid nanoparticles may be formulated with various adjuvants, fragrances and dyes, as well as fats and oils, surfactants, humectants, pH conditioners, thickeners, preservatives, antioxidants, UV-absorbing agents, pigments, cleansing agents, desiccating agents, emulsifiers, and other ingredients commonly used in cosmetic products.

The composition of the present invention may be applied to medical instruments such as stents and cannulas.

The present invention will now be described in detail with reference to several Test Examples.

As specific examples of the retinoic acid nanoparticles of the present invention coated with an inorganic salt of polyvalent metal, the calcium carbonate-coated retinoic acid nanoparticles may be referred to as "retinoic acid-CaCO₃ nanoparticles," the zinc carbonate-coated retinoic acid nanoparticles may be referred to as "retinoic acid-ZnCO₃ nanoparticles," and the calcium phosphate-coated retinoic acid nanoparticles may be referred to as "retinoic acid-CaPO₄ nanoparticles."

### Test Example 1: Preparation of retinoic acid-CaCO₃ nanoparticles

13.6mg retinoic acid was dissolved in 900µl ethanol (or methanol). To this solution, 100µl of 0.5N aqueous NaOH solution were added. The pH of the mixture was 7 to 7.5. A 100µl portion of the mixture was then added to 100µl distilled water containing Tween 80 and the resulting mixture was thoroughly stirred.

After approximately 30 min, a 5M aqueous calcium chloride solution was added and the mixture was stirred for another 30 min. Subsequently, a 1M aqueous sodium carbonate solution was added and the mixture was further stirred. After continuous stirring over one day and night, the solution was freeze-dried over night to give desired calcium carbonate-coated retinoic acid nanoparticles.

This process was repeated by varying the molar ratio of calcium chloride and sodium carbonate added to the retinoic acid micelles to produce retinoic acid-CaCO₃ nanoparticles. The nanoparticles were then ultrasonicated for 5min. The particle size (diameter) of the resultant nanoparticles immediately after preparation and after the sultraonication is shown in Table 1 below for each molar ratio.

**Table 1 The effect of the molar ratio of calcium chloride to sodium carbonate on the particle size of nanoparticles**

| | Average particle size of retinoic acid-CaCO₃ nanoparticles | | | | | | |
|---|---|---|---|---|---|---|---|
| Molar ratio of CaCl₂/NaCO₃ | 1/0 1/0 | 1/0.01 1/0.01 | 1/0.1 1/0.1 | 1/0.2 1/0.2 | 1/0.3 1/0.3 | 1/0.5 1/0.5 | 1/1.0 1/1. 0 |
| Average particle size immediately after preparation | 20.2 | 23 | 17.3 | 30.3 | 356.3 | 1316. 2 | 1450 |
| Average particle size after ultrasonication after ultrasonication | - | - | - | 22.4 | 33.3 | 41.1 | 106.4 |

As can be seen from the results of Table 1, by varying the molar ratio of calcium chloride and sodium carbonate added to the retinoic acid micelles, the particle size of the resulting retinoic acid-CaCO₃ nanoparticles can be adjusted.

Specifically, by adjusting the molar ratio of calcium chloride to sodium carbonate to 1:0 to 1:0.2, the calcium carbonate coating is deposited on the surface of the retinoic acid micelles to form nanoparticles sized 10 to 50 nm.

When the molar ratio of calcium chloride to sodium carbonate was in the range of 1:0.3 to 1:1.0, the average particle size of the nanoparticles immediately after the preparation was approximately 350 to 1500 nm. This indicates that the fine nanoparticles aggregated into large clusters with large average particle size.

These clusters can be dispersed by ultrasonication into highly uniform nanoparticles with an average particle size of approximately 100 nm.

Thus, it has been proven that by adding calcium chloride and sodium carbonate so that the molar ratio of the former to the latter is 1:0 to 1:1.0, and mechanically shaking the resulting particles by for example ultrasonication, the size of the resulting retinoic acid-CaCO₃ nanoparticles can be adjusted to a range of 5 to 300 nm.

For testing, the freeze-dried retinoic acid-CaCO₃ nanoparticles were dispersed again in injectable distilled water to a predetermined concentration.

### Test Example 2: Preparation of retinoic acid-ZnCO₃ nanoparticles

13.6mg retinoic acid was dissolved in 900µl ethanol. To this solution, 100µl of 0.5N aqueous NaOH solution were added. The pH of the mixture was 7 to 7.5. A 100µl portion of the mixture was then added to 100µl distilled water containing Tween 80 and the resulting mixture was thoroughly stirred.

After approximately 30 min, a 5M aqueous zinc acetate solution was added and the mixture was stirred for another 30 min. Subsequently, a 1M aqueous sodium carbonate solution was added and the mixture was further stirred. The mixture was continuously stirred over one day and night, and the resulting solution was freeze-dried over night to give desired zinc carbonate-coated retinoic acid nanoparticles (retinoic acid-ZnCO₃ nanoparticles).

The resulting retinoic acid-ZnCO₃ nanoparticles were similar to the nanoparticles of Test Examples 1 in terms of particle size distribution.

For testing, the freeze-dried retinoic acid-ZnCO₃ nanoparticles were dispersed again in injectable distilled water to a predetermined concentration.

### Test Example 3: Preparation of retinoic acid-CaPO₄ nanoparticles

13.6mg retinoic acid was dissolved in 900µl ethanol. To this solution, 100µl of 0.5N aqueous NaOH solution were added. The pH of the mixture was 7 to 7.5. A 100µl portion of the mixture was then added to 100µl distilled water containing Tween 80 and the resulting mixture was thoroughly stirred.

After approximately 30 min, a 5M aqueous calcium chloride solution was added and the mixture was stirred for another 30 min. Subsequently, a 1M aqueous sodium phosphate solution was added and the mixture was further stirred. The mixture was continuously stirred over one day and night, and the resulting solution was freeze-dried over night to give desired calcium phosphate-coated retinoic acid nanoparticles (retinoic acid-CaPO₄ nanoparticles).

The resulting retinoic acid-CaPO₄ nanoparticles were also similar to the nanoparticles of Test Example 1 in terms of particle size distribution.

The retinoic acid nanoparticles coated with an inorganic salt of polyvalent metal thus obtained were analyzed in the following biological tests for their pharmacological activity and the effect of their particle size on the activity.

### Test Example 4: in vitro experiment to determine the effect of CaCO₃ nanoparticles on B16 melanoma cells

It is a well-known fact that retinoic acid has an ability to suppress the growth of B16 melanoma cells. The following tests were conducted to determine if the growth of B16 melanoma cells could be suppressed by the retinoic acid-CaCO₃ nanoparticles obtained in the above-described Test Examples and how significant the suppressive effect would be as compared to non-nanoparticle retinoic acid alone.

### (Method)

B16 melanoma cells (2 x 10⁴ cells) were cultured in separate wells for 24 hours. To these wells, retinoic acid or the retinoic acid-CaCO₃ nanoparticles were added at different concentrations and the cells were cultured for additional 48 hours. Subsequently, the uptake of ³H-thymidine by the cells was measured for each well and the DNA synthesis was compared between the cells.

### (Results)

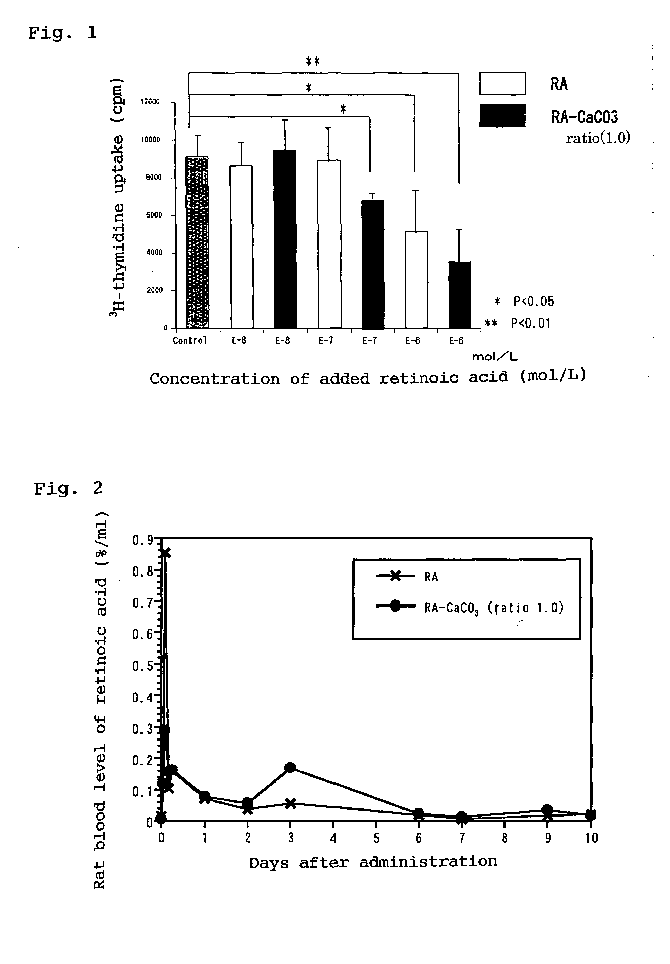

The results are shown in Fig. 1. The results indicate that the retinoic acid-CaCO₃ nanoparticles of the present invention show higher growth inhibition than non-nanoparticle retinoic acid alone with the difference becoming more significant at higher concentrations.

Thus, the retinoic acid-CaCO₃ nanoparticles of the present invention have been proved to be highly effective in the suppression of the growth of B16 melanoma cells.

### Test Example 5: in vivo experiment to determine kinetics of subcutaneously administered nanoparticles in blood in rats

### (1) Subcutaneous administration

### (Method)

³H-labelled retinoic acid and retinoic acid-CaCO₃ nanoparticles obtained from ³H-labelled retinoic acid micelles were subcutaneously administered to Wistar rats (7 week old/male). Blood samples were collected at intervals and analyzed for the retinoic acid level using a scintillation counter.

The retinoic acid-CaCO₃ nanoparticles used were obtained by adding to the retinoic acid micelles calcium chloride and sodium carbonate at a molar ratio of 1:1.0 (average particle size = 150 nm).

As a control, retinoic acid micelles were used without being formed into nanoparticles.

### (Results)

Fig. 2 shows the comparison of the effect of subcutaneous administration between the retinoic acid-CaCO₃ nanoparticles (average particle size = approx. 150 nm) and the non-nanoparticle retinoic acid micelles to serve as control.

As can be seen from the results, the non-nanoparticle retinoic acid micelles to serve as control released significant amounts of retinoic acid within about 1 hour after administration, whereas the blood level of retinoic acid was initially kept low and the release of retinoic acid was sustained over about 7 days period for the retinoic acid-CaCO₃ nanoparticles.

These results support the ability of the retinoic acid-CaCO₃ nanoparticles to release retinoic acid in a sustained manner and thus prove the effectiveness of the nanoparticles as a sustained-release preparation.

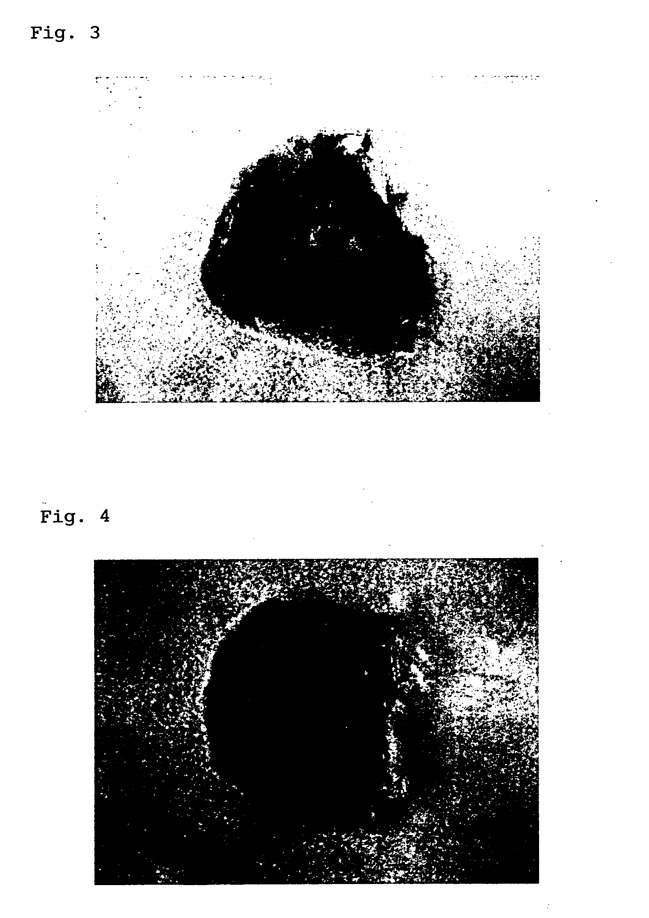

Figs. 3 and 4 are photographs of the site of application 10 days after administration of the non-nanoparticle retinoic acid micelles as control (Fig. 3) and the retinoic acid-CaCO₃ nanoparticles (Fig. 4). It is seen that the irritancy of retinoic acid caused inflammation at the site of application after the administration of the retinoic acid micelles, whereas no inflammation was induced after the administration of the retinoic acid-CaCO₃ nanoparticles, indicating reduced irritancy of retinoic acid.

These results indicate that the retinoic acid-CaCO₃ nanoparticles to serve as the active ingredient of the present invention have reduced skin irritancy and are therefore safe for use in external applications or cosmetics for skin application.

### (2) Topical application

The dorsal skin of mice (ddy strain/5week old/male) was clipped with electric clippers, and the ³H-labelled retinoic acid, as well as the retinoic acid-CaCO₃ nanoparticles and the retinoic acid-ZnCO₃ nanoparticles obtained from the ³H-labelled retinoic acid micelles, was mixed with a Vaseline base and was applied to the clipped area. Blood samples were collected at intervals and analyzed for the retinoic acid level using a scintillation counter.

The samples tested were as follows:
(a) Retinoic acid-CaCO₃ nanoparticles (average particle size = approx. 20 nm); and
(b) Retinoic acid-ZnCO₃ nanoparticles (average particle size = approx. 20 nm).

Retinoic acid that was not formed into nanoparticles was mixed with a Vaseline base and used as control.

### (Results)

The results are shown in Table 5. As shown, the blood level of retinoic acid was significantly higher after topical application of the retinoic acid-CaCO₃ nanoparticles (RA- CaCO₃) (average particle diameter = approx. 20 nm) or the retinoic acid-ZnCO₃ nanoparticles (RA-ZnCO₃) (average particle diameter = approx. 20 nm) mixed with Vaseline base than after topical application of the non-nanoparticle retinoic acid.

Accordingly, it has been demonstrated that the pharmacological effect of the retinoic acid nanoparticles to serve as the active ingredient of the present invention can be significantly enhanced by adjusting the average particle size to make very small nanoparticles.

### Test Example 6: in vivo experiment to determine expression levels of HB-EGF m-RNA in mice

### (Method)

A Vaseline-based retinoic acid preparation (containing 0.1% retinoic acid) was applied to the pinna of the ear of mice (ddy strain, 5 week old/male) 30mg/penna/day for 4 consecutive days. The ear was excised on Day 5 and RNA was extracted using real-time PCR to determine the expression level of HB-EGF m-RNA.

Glyceraldehyde-3-phosphate dehydrogenase (GAPDH) m-RNA was also synthesized as a housekeeping gene and quantified to serve as a standard.

The retinoic acid samples tested were as follows:
(a) Retinoic acid-CaCO₃ nanoparticles (average particle size = approx. 20 nm);
(b) Retinoic acid-ZnCO₃ nanoparticles (average particle size = approx. 20 nm); and
(c) Retinoic acid alone.

### (Results)

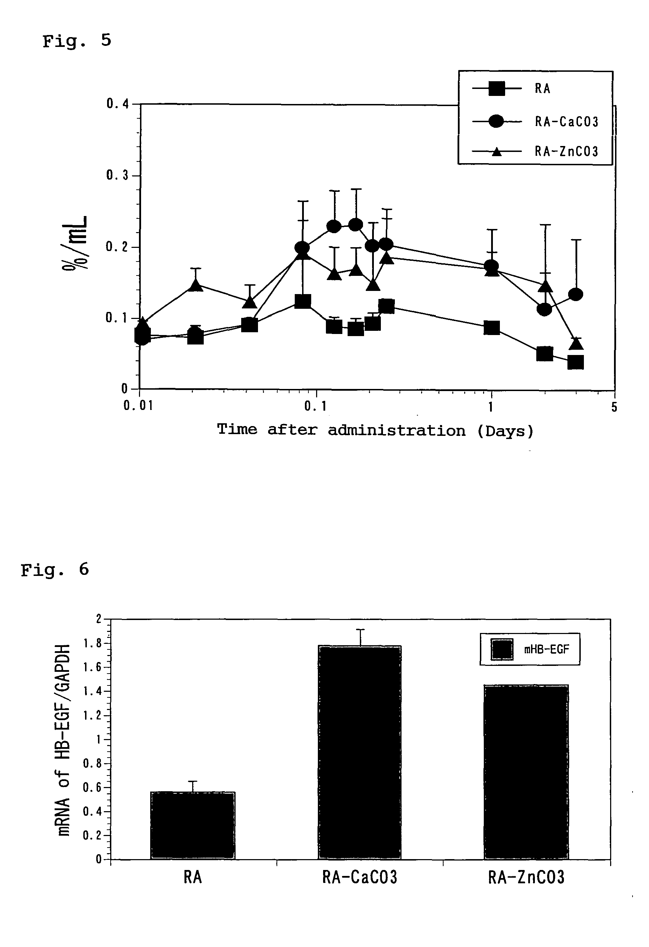

The results are shown in Fig. 6. As shown, the expression level of HB-EGF m-RNA was significantly higher for the size-adjusted retinoic acid-CaCO₃ nanoparticles (average particle size = approx. 20 nm) and the retinoic acid-ZnCO₃ nanoparticles (average particle size = approx. 20 nm) of the present invention than for retinoic acid alone.

The fact that expression of mRNA of HB-EGF, an epithelial growth factor, was enhanced by the retinoic acid nanoparticles coated with an inorganic acid of polyvalent metal of the present invention suggests that the nanoparticles have high ability to facilitate the skin regeneration.

### Test Example 7: in vivo experiment to determine the effect of topical application of nanoparticles on the growth of epithelial cells in mice

### (Method)

The dorsal skin of mice (ddy strain/5week old/male) was clipped with electric clippers and a Vaseline based retinoic acid preparation (containing 0.1% retinoic acid) was applied to the clipped area 10mg/cm² per day for 4 consecutive days. The epithelial thickness at the site of application was measured on Day 4.

The retinoic acid samples tested were as follows:
(a) Retinoic acid-CaCO₃ nanoparticles (average particle size = approx. 20 nm);
(b) Retinoic acid-ZnCO₃ nanoparticles (average particle size = approx. 20 nm);
(c) Retinoic acid-Ca nanoparticles obtained by adding calcium chloride to retinoic acid micelles to deposit calcium coating on the micelle surface (average particle size = approx. 20 nm);
(d) Retinoic acid-Zn nanoparticles obtained by adding zinc chloride to retinoic acid micelles to deposit zinc coating on the micelle surface (average particle size = approx. 20 nm); and
(e) Retinoic acid alone.

As controls, one group was given no treatment and one group was given Vaseline alone.

### (Results)

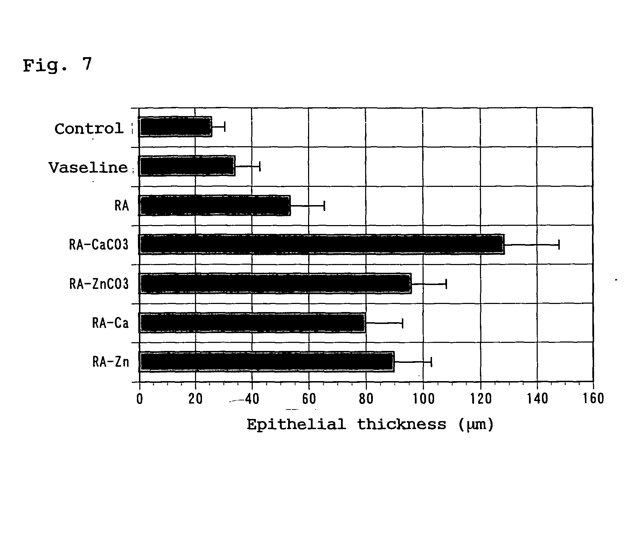

The results are shown in Fig. 7. As shown, the growth of epithelial cells was significantly faster and the increase in the epithelial thickness was significantly greater for the retinoic acid-CaCO₃ nanoparticles and the retinoic acid-ZnCO₃ nanoparticles than for the retinoic acid preparation.

The increase in the epithelial thickness was also greater for the retinoic acid-Ca nanoparticles (Ra-Ca), obtained by adding calcium chloride to retinoic acid micelles to deposit calcium coating on the micelle surface, and for the retinoic acid-Zn nanoparticles (RA-Zn), obtained by adding zinc chloride to retinoic acid micelles to deposit zinc chloride coating on the micelle surface, than for retinoic acid alone. This suggests that the retinoic acid micelles stabilized by metal halide or metal acetate coating can significantly facilitate the growth of epithelial cells as compared to the retinoic acid preparation. Such coated micelles are also encompassed by the scope of the present invention.

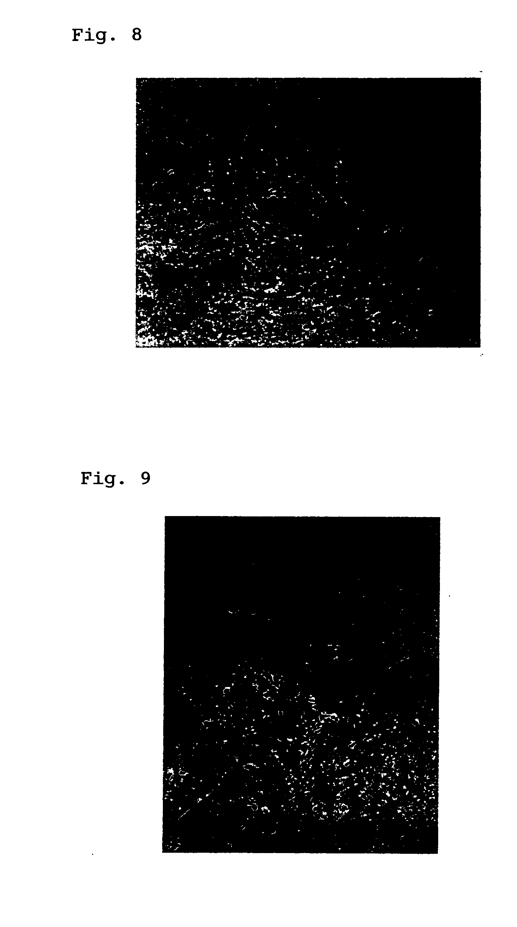

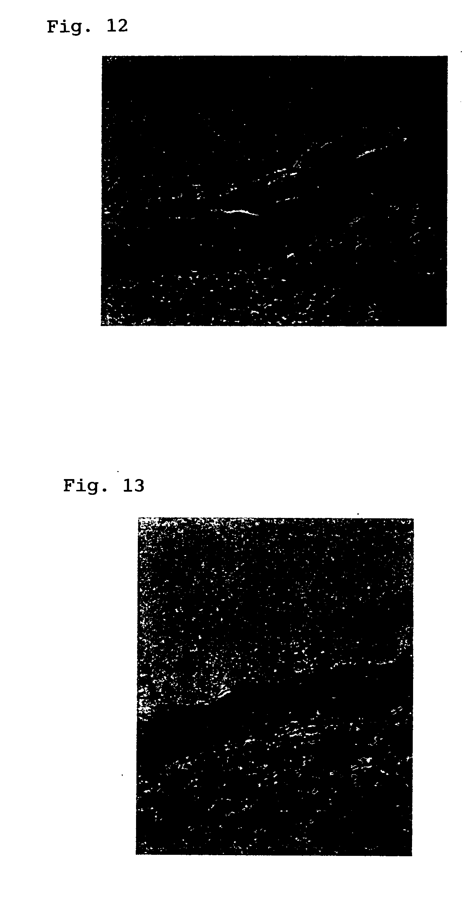

Figs. 8 through 13 are photographs of HE-stained skin tissues, where Fig. 8 corresponds to the non-treated group to serve as control; Fig. 9 to the group administered the preparation containing retinoic acid alone; Fig. 10 to the group administered the preparation containing retinoic acid-CaCO₃ nanoparticles; Fig. 11 to the group administered the preparation containing retinoic acid-ZnCO₃ nanoparticles; Fig. 12 to the group administered retinoic acid-Ca nanoparticles; and Fig. 13 to the group administered retinoic acid-Zn nanoparticles. The area stained purple in these photographs is the epithelium.

The photographs indicate that the regeneration of the epithelium was significantly facilitated in the group given the retinoic acid nanoparticles coated with an inorganic acid of polyvalent metal of the present invention as compared to the control or the group given the preparation containing retinoic acid alone.

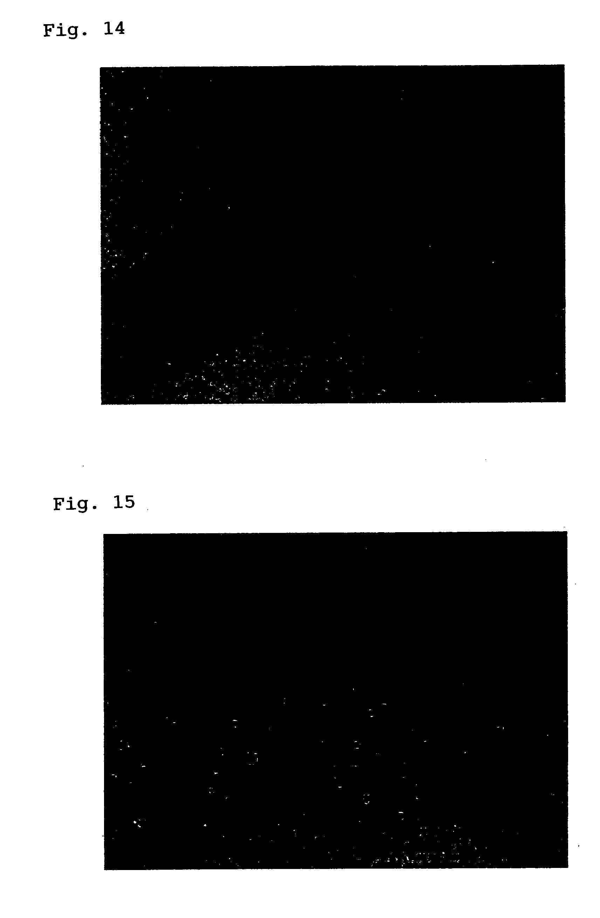

Figs. 14 and 15 are photographs of skin tissue stained with colloidal iron, where Fig. 14 corresponds to the group administered the preparation containing retinoic acid alone; and Fig. 15 corresponds to the group administered the preparation containing the retinoic acid-CaCO₃ nanoparticles. The photographs indicate that the basal cells and prickle cells of the epithelium are stained dark blue in the skin tissue to which the preparation containing the retinoic acid-CaCO₃ nanoparticles of the present invention has been applied, indicating the production of hyaluronic acid in these cells. The staining is less significant in the skin tissue to which the preparation containing retinoic acid alone has been applied. This suggests that transdermal administration of the retinoic acid-CaCO₃ nanoparticles of the present invention quickly induces the production of hyaluronic acid, which otherwise is not produced in the epithelium in a short period of time.

### Test Example 8: in vivo experiment to determine the ability of the nanoparticles to eliminate wrinkles in hairless mice

### (Method)

A Vaseline-based preparation containing the retinoic acid-CaCO₃ nanoparticles of the present invention (average particle size = 20 nm) was applied to the heavily wrinkled neck area of hairless mice at a dose of 30 mg/day/neck for 4 consecutive days. The degree of skin elimination was observed on Day 4.

As control, one group was given a preparation containing retinoic acid alone.

### (Results)

The results are shown in Figs. 16 and 17, where Fig 16 is a photograph of the neck area prior to application of the preparation; and top picture in Fig. 17 is of the neck area after application of the preparation containing retinoic acid alone, and bottom picture is of the neck area after application of the preparation containing retinoic acid-CaCO₃ nanoparticles of the present invention. The comparison between these pictures demonstrates that the deep skin wrinkles initially observed in the neck area have been eliminated by the application of the preparation of the present invention, resulting in smooth skin.

### Test Example 9: Test for storage stability of the preparation of the present invention

To determine the storage stability of the preparation that contains the retinoic acid-CaCO₃ nanoparticles (average particle size = 20 nm) of the present invention as an active ingredient, a Vaseline-based preparation (with 0.1% retinoic acid) and a water-based preparation were stored at 37°C and were compared for the degree of retinoic acid degradation.

A Vaseline-based preparation containing retinoic acid alone (0.1% retinoic acid) was prepared as control and was also stored.

Using a spectrophotometer, the absorption by each preparation was measured 10, 24, and 46 days after the start of the storage period, and the change in the peak absorption (340 nm) was compared.

### (Results)

The results are shown in Figs. 18 though 20, where Fig. 18 shows the change in the peak absorption by the preparation containing retinoic acid alone; Fig. 19 shows the change in the peak absorption by the aqueous preparation containing the retinoic acid-CaCO₃ nanoparticles of the present invention; and Fig. 20 shows the change in the peak absorption by the Vaseline-based preparation containing the retinoic acid-CaCO₃ nanoparticles of the present invention.

The comparison between the three preparations indicates that retinoic acid remains highly stable in the preparations of the present invention.

Exemplary preparations that use the retinoic acid nanoparticles coated with an inorganic salt of polyvalent metal of the present invention will now be presented. It should be appreciated that these preparations are described by way of example only and are not exhaustive.

The retinoic acid nanoparticles coated with an inorganic salt of polyvalent metal used in the following preparation examples were obtained by dispersing freeze-dried nanoparticles again in distilled water to a predetermined concentration.

### Preparation Example 1: External Ointment/Hydrogel

Predetermined amounts of retinoic acid-CaCO₃ nanoparticles (average particle size = approx. 20 nm), white Vaseline, carboxymethylcellulose, and methyl paraoxybenzoate were mixed together until uniform to form an ointment containing 0.3% retinoic acid. In the same manner, a hydrogel containing 0.3% retinoic acid was also obtained.

**Preparation Example 2: External Patch (Aqueous Poultice)**

| (Formula) | |
|---|---|
| Retinoic acid-CaCO₃ nanoparticles (or retinoic acid-ZnCO₃ nanoparticles) | 0.1 wt% |
| Polyacrylic acid | 2.0 wt% |
| Sodium polyacrylate | 5.0 wt% |
| Sodium carboxymethylcellulose | 2.0 wt% |
| Gelatin | 2.0 wt% |
| Polyvinyl alcohol | 0.5 wt% |
| Glycerol | 25.0 wt% |
| Kaolin | 1.0 wt% |
| Sodium hydroxide | 0.6 wt% |
| Tartaric acid | 0.3 wt% |
| EDTA-2-sodium | 0.1 wt% |
| Purified water | Remainder |

The components listed above were mixed together by a known technique to prepare an external patch (aqueous poultice).

### Preparation Example 3: Cosmetic Cream

1 part of carboxyvinyl polymer was dissolved in 89 parts purified water. To this solution, 0.4 parts sodium hydroxide in 9.6 parts purified water were added to form 100 parts of an alkali-neutralized aqueous gel of calboxyvinyl polymer.

Using a known technique, the aqueous gel was then used to prepare a cosmetic cream having the following formula.

| | |
|---|---|
| Retinoic acid-CaCO₃ nanoparticles | 0.1 parts |
| Aqueous gel | 30 parts |
| Liquid paraffin | 10 parts |
| Glycerol | 22.7 parts |
| Methyl paraoxybenzoate | 0.3 parts |
| Stearic acid | 10 parts |
| Fragrance | Proper amount |
| Purified water | Remainder |

**Preparation Example 4: Cosmetic Emulsion**

| (Formula) | |
|---|---|
| Cetyl alcohol | 1.5 parts |
| Vaseline | 5.0 parts |
| Liquid paraffin | 10.0 Parts |
| Polyoxyethylene(10)sorbitan monostearate | 10.0 parts |
| Polyexyethylene glycol (1500) | 3.0 parts |
| Triethanolamine | 1.0 parts |
| Tocopherol acetate | 0.2 parts |
| Retinoic acid-CaCO₃ nanoparticles (or retinoic acid-ZnCO₃ nanoparticles) | 0.05 parts |
| Sodium hydrogen sulfite | 0.01 parts |
| Carboxyvinyl polymer (Trade name: Hibiswako) | |
| | 0.05 parts |
| Methyl paraben | Proper amount |
| Fragrance | Proper amount |
| Purified water | Remainder |

### (Production Process)

Carboxyvinyl polymer is dissolved in a small portion of purified water (Solution A). Polyethyleneglycol, retinoic acid-CaCO₃ nanoparticles (or retinoic acid-ZnCO₃ nanoparticles), and triethanolamine are dissolved in the remainder of purified water and the solution is heated and kept at 70°C (Aqueous phase). The other components are mixed together and the mixture is kept at 70°C (Organic phase). The organic phase and Solution A are sequentially added to the aqueous phase. The mixture is uniformly emulsified while kept at the same temperature. Once emulsified, the emulsion is cooled while being stirred. This gives the desired cosmetic emulsion.

### INDUSTRIAL APPLICABILITY

As set forth, the present invention provides a composition that contains as an active ingredient nanoparticles comprising retinoic acid micelles with a coating of polyvalent metal inorganic salt deposited on the surface thereof. The retinoic acid nanoparticles coated with an inorganic salt of polyvalent metal to serve as the active ingredient of the present invention can be dissolved in water to make a stable clear solution that can be formulated into injectable preparations for subcutaneous and intravenous administration. The polyvalent metal inorganic salt coating helps reduce the irritancy of retinoic acid, so that the nanoparticles do not cause inflammation or tumor formation at the site of application.

Effectively making use of the pharmacological effect of retinoic acid, the retinoic acid nanoparticles coated with an inorganic salt of polyvalent metal of the present invention are expected to find wide applications in oral preparations, non-oral preparations, external preparations, and cosmetics and are thus of significant industrial importance.

## Claims

1. A composition comprising as an active ingredient retinoic acid nanoparticles comprising micelles of retinoic acid coated with an inorganic salt of polyvalent metal and having an average particle size of 5 to 300 nm.

2. The composition according to claim 1, wherein a coating of the polyvalent metal inorganic salt of the retinoic acid nanoparticles to serve as the active ingredient is calcium carbonate, zinc carbonate, or calcium phosphate.

3. The composition according to claim 1 or 2, wherein the retinoic acid nanoparticles to serve as the active ingredient is obtained by:
dispersing retinoic acid dissolved in a lower alcohol in an aqueous alkali solution;
adding a nonionic surfactant to the dispersion to form a mixed micelle;
adding to the micelle a halide or acetate of divalent metal along with a carbonate or phosphate of alkali metal so that a molar ratio of the former to the latter is 1:0 to 1:1.0, thereby depositing a coating of the inorganic salt of the polyvalent metal on a surface of the micelle; and
adjusting an average particle size of the resulting nanoparticles to 5 to 300 nm.

4. The composition according to claim 1, 2, or 3, wherein the active ingredient is retinoic acid nanoparticles having an average particle size of 5 to 300 nm and coated with calcium carbonate.

5. The composition according to claim 1, 2, or 3, wherein the active ingredient is retinoic acid nanoparticles having an average particle size of 5 to 300 nm and coated with zinc carbonate.

6. The composition according to claim 1, 2, or 3, wherein the active ingredient is retinoic acid nanoparticles having an average particle size of 5 to 300 nm and coated with calcium phosphate.

7. The composition according to any of claims 1 to 6 for use as an oral preparation, a non-oral preparation, an external preparation, or a cosmetic.

8. The composition according to claim 7 being a sustained-release composition.

9. A sustained-release preparation containing as an active ingredient retinoic acid nanoparticles having an average particle size of 5 to 300 nm and coated with calcium carbonate.

10. An external preparation containing as an active ingredient retinoic acid nanoparticles having an average particle size of 5 to 300 nm and coated with calcium carbonate.

11. A cosmetic containing retinoic acid nanoparticles having an average particle size of 5 to 300 nm and coated with calcium carbonate.

12. A sustained-release preparation containing as an active ingredient retinoic acid nanoparticles having an average particle size of 5 to 300 nm and coated with zinc carbonate.

13. An external preparation containing as an active ingredient retinoic acid nanoparticles having an average particle size of 5 to 300 nm and coated with zinc carbonate.

14. A cosmetic containing retinoic acid nanoparticles having an average particle size of 5 to 300 nm and coated with zinc carbonate.

15. A sustained-release preparation containing as an active ingredient retinoic acid nanoparticles having an average particle size of 5 to 300 nm and coated with calcium phosphate.

16. An external preparation containing as an active ingredient retinoic acid nanoparticles having an average particle size of 5 to 300 nm and coated with calcium phosphate.

17. A cosmetic containing retinoic acid nanoparticles having an average particle size of 5 to 300 nm and coated with calcium phosphate.
